Europäisches Patentamt

European Patent Office

Office européen des brevets

(19)

(11) Publication number: **0 382 441**
**A2**

# EUROPEAN PATENT APPLICATION

(12)

(21) Application number: 90301162.5

(22) Date of filing: 05.02.90

(51) Int. Cl.⁵: **C07C 315/02, C07C 315/06,**
**C07C 317/14**

(30) Priority: 09.02.89 GB 8902915

(43) Date of publication of application:
16.08.90 Bulletin 90/33

(84) Designated Contracting States:
AT BE CH DE DK ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Atherton, John Heathcote
15 Prestwich Drive
Fixby Drive, Huddersfield(GB)
Inventor: Griffin, James Benedict
Fur-tree Cottage, Cooper Lane, Potto Village
Northerallerton, North Yorkshire(GB)
Inventor: Fawkner, Robert Douglas
Sweet, Briarhazelwood Close
Bexill on Sea, East Sussex(GB)
Inventor: Hodgkinson, Ian
1 Bankfield Drive
Holmbridge, Huddersfield(GB)

(74) Representative: Chapman, Kenneth Hazel et al
Imperial Chemical Industries PLC Legal
Department: Patents P.O. Box 6 Bessemer
Road
Welwyn Garden City Herts, AL71HD(GB)

(54) Production of aromatic sulphones.

(57) A process for the production of aromatic sulphones involves providing a substantially anhydrous starting melt in which the relative concentrations of sulphonic acid and sulphone are on the sulphone-rich side of the relevant eutectic in the sulphonic acid/sulphone phase diagram. The sulphone is purified by fractional melt crystallisation.

Fig. 1(b)

EP 0 382 441 A2

EP 0 382 441 A2

## Production of Aromatic Sulphones

This invention relates to the production of aromatic sulphones and in particular to a step of recovering an aromatic sulphone from a mixture containing also an aromatic sulphonic acid and a manufacturing process based on that step.

According to the invention a process for producing an aromatic sulphone by reacting a sulphonic acid with an aromatic compound having at least one reactive position and recovering the sulphone from the reaction mixture is characterised by

a) providing a substantially anhydrous starting melt, optionally containing at most 18% w/w of volatile solvent, in which the relative concentrations of sulphonic acid and sulphone are on the sulphone-rich side of the relevant eutectic in the sulphonic acid/sulphone phase diagram;

b) cooling the melt to a temperature at which the sulphone crystallises and allowing it to crystallise;

c) separating the sulphone crystals and running off the supernatant liquid.

This process is carried out preferably in combination with the step of providing the starting melt by reacting the sulphonic acid with the aromatic compound in water-removing conditions and controlling the reaction so that the relative concentrations of the unreacted sulphonic acid and sulphone are as defined in step (a).

The invention includes also the combination process comprising also producing the sulphonic acid by sulphonating the aromatic compound at a temperature preferably below $220^\circ$ C and especially between 80 and $215^\circ$ C. Examples of preferred sulphonating agents are mentioned below.

Preferably the supernatant liquid is at least partly recycled to the reaction of the sulphonic acid with the aromatic compound, whereby to utilise unreacted sulphonic acid and possibly also to re-equilibrate sulphone isomers if more than one is present. Recycle to the initial sulphonation is usable but less desirable.

For further purification of sulphone, the process can include at least once remelting the crystals from step (c) and subjecting the melt to a further succession in cascade of steps (b) and (c), followed preferably by recycling the supernatant liquid.

Each crystallisation is preferably carried out suitably by flowing the melt over a cooled surface, so that the crystals adhere to the surface, and periodically removing those crystals. The surface can be that of tubes of a tube-in-shell heat exchanger, preferably on the inside of the tubes, if removal is to be mechanical. More conveniently removal is by remelting, especially if there is a further step of crystallisation, since the resulting melt can be fed direct to the further heat exchanger in which the next cooling step is to take place. Preferably such remelting is carried out at least 2 staged temperatures: in the stages at relatively low temperatures lower-melting materials are taken off: and in the stage at highest temperature relatively pure required sulphone is recovered. In the particular process for making 4,4' - dichlorodiphenylsulphone (4,4'-DCDPS), the earlier remelt stages ("sweating") are at temperatures from the eutectic up to the melting point of 4,4'-DCDPS, typically in the range 30-140$^\circ$ C, especially 65-140$^\circ$ C. Fractions rich in 4-chlorobenzenesulphonic acid, the 2,4' and 3,4' sulphone isomers are produced by each sweating.

The process is especially useful for making sulphones in which both groups linked to $SO_2$ are unsubstituted phenyl or phenyl carrying one or both of the positions para to the $SO_2$ an ortho-para directing substituent, for example halogen, $C_1$-$C_3$ alkyl, $C_1$-$C_3$ alkoxy or phenyl. Such para substitution commonly goes with relatively high melting point; but the formation of the required sulphone may be accompanied by side reactions to sulphones in which one or both substituents is ortho to $SO_2$. Such sulphones, being low-melting, remain in the liquid phase when the wanted sulphone crystallises. They can be recovered if there is a use for them or, if they undergo isomerisation, can be treated appropriately, especially by recycle.

The invention is illustrated by the accompanying drawings in which:

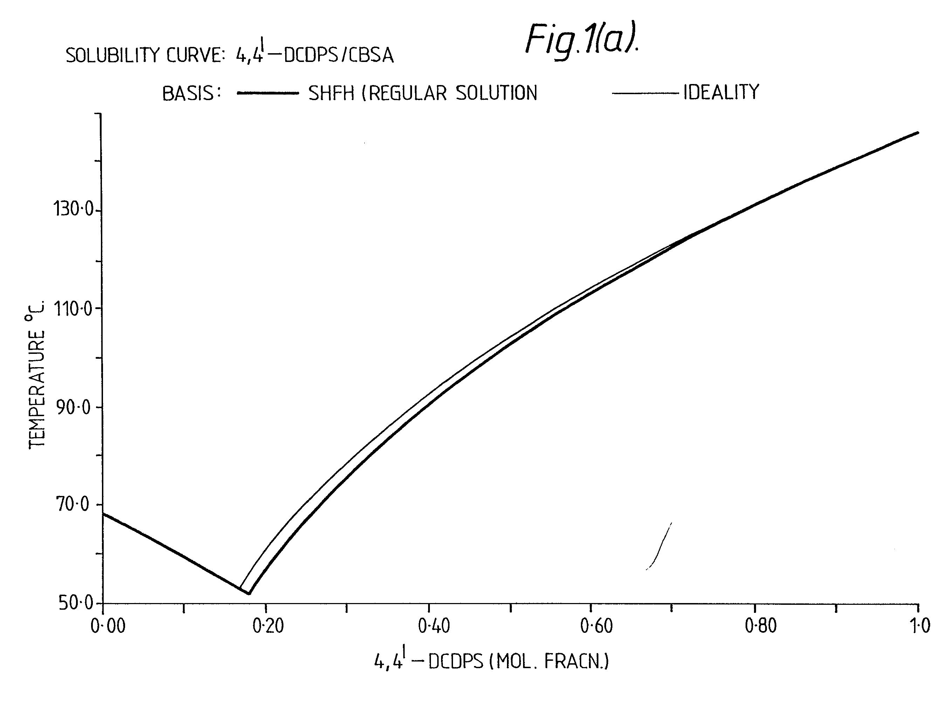

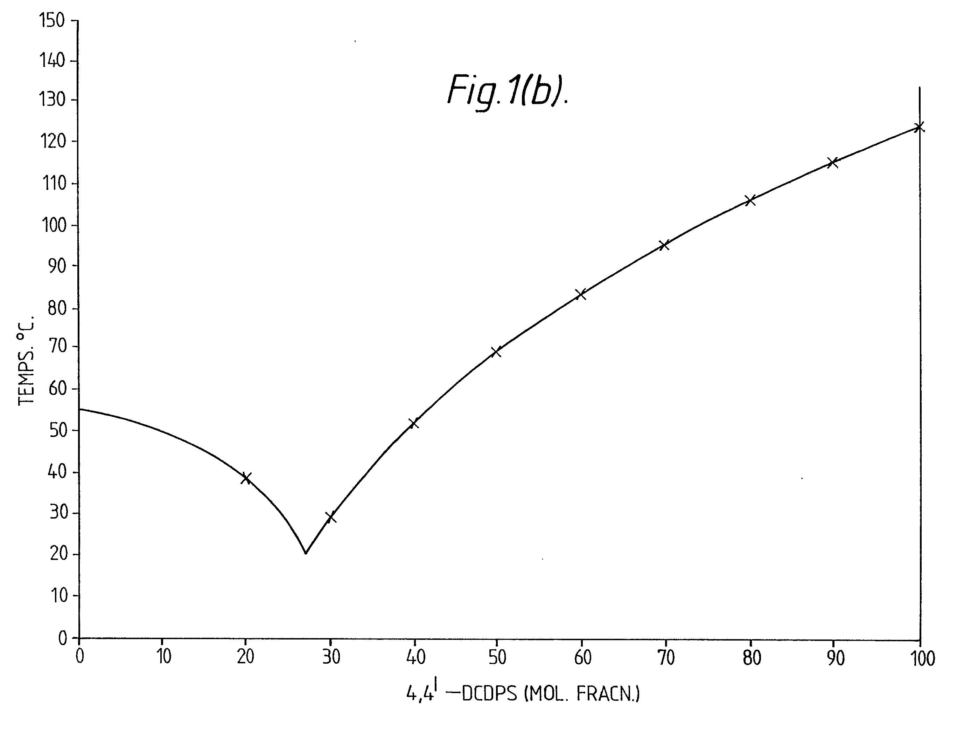

Figure 1 (a) shows a calculated phase diagram for mixtures of 4,4'-dichlorodiphenylsulphone and chlorobenzene-4-sulphonic acid (CBSA). This is based on the following assumptions:

i) No solid solutions are formed

ii) No polymorphic forms are exhibited

iii) No association in the liquid phase

iv) Non-polar components.

Figure 1 (b) shows the phase diagram as determined experimentally.

Whereas at the time of filing the priority application it was thought possible that CBSA and DCDPS would associate by hydrogen-bonding in the liquid phase, the experimentally-determined diagram shows that this is not so. The calculated and experimental diagrams differ as follows

2

| | Calculated | Experimental |
|---|---|---|
| Eutectic temperature °C | 53 | 20 |
| DCDPS mol fraction | 0.18 | 0.24 |

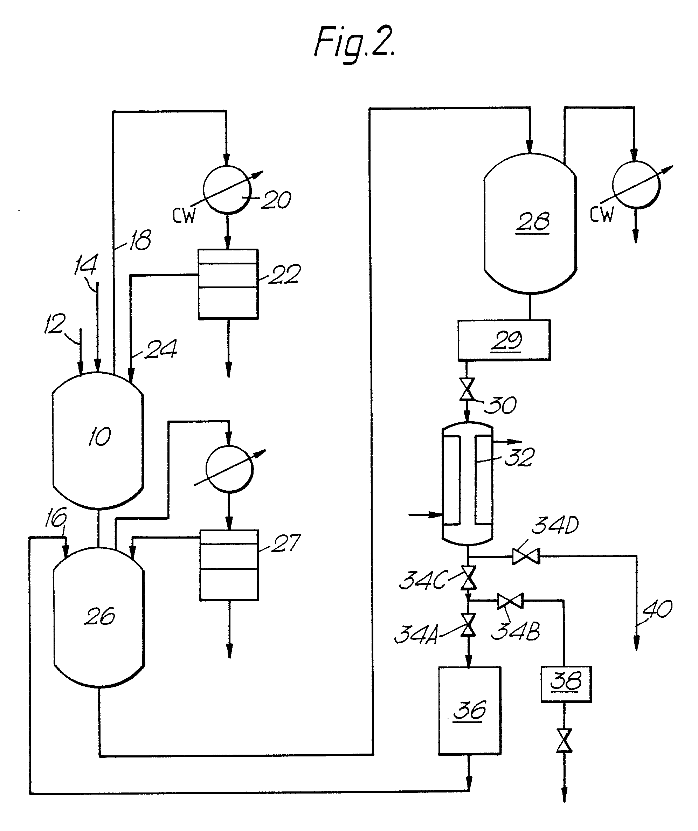

Figure 2 shows a flow sheet of a process according to the invention.

Referring to figures 1 (a) and (b) it is evident that a eutectic occurs at about 24 mol% of DCDPS, and thus that any molten mixture containing more than that percentage of 4,4'-DCDPS will on cooling be resolved into solid DCDPS and a liquid phase richer in CBSA than the starting mixture. For adequate over-all efficiency the content of 4,4'-DCDPS in the starting mixture is preferably substantially greater than the eutectic concentration.

Referring to figure 2, reactor 10 is fed at 12 with monochlorobenzene (MCB), at 14 with oleum. Reactor 10 is heated if necessary and maintained at near the boiling point of MCB in the reaction mixture. The vapour is taken off at 18 and condensed at 20, to give a liquid which is phase-separated at 22 into water (which is discarded) and MCB (which is returned at 24). The rate at which water is discarded from 22 depends on the sulphur trioxide concentration in the oleum fed at 14. It would also be possible to use sulphuric acid, in which event the rate of water discard would be greater, or substantially pure sulphur trioxide, in which event the rate of water discard would be very low. At the temperature of reactor 10 substantially complete conversion of sulphonating agent to CBSA is effected, but little further reaction to DGDPS occurs.

The product of reactor 10 is in reactor 26 mixed with recycled liquid fed at 16 from tank 36 to be described and reacted with reflux of MCB until its content of 4,4'-DCDPS exceeds the eutectic shown in figure 1(b). The refluxing MCB is condensed and the condensate is separated at 27 into water (which is discarded) and MCB, which is returned to reactor 26. The water discard is substantially the whole of that produced by the sulphone formation, plus any that entered with the product of reactor 10 then passed to reactor 28 in which some unreacted MCB is taken off to give a melt; the MCB content of the product having 28 is typically 5-15% by weight. The melt is fed to buffer tank 29 and thereafter to falling-film crystalliser 30, in which a solid rich in 4,4'-DCDPS is deposited in the inside surfaces of coolant-surrounded tubes 32 and, with valve 34A and 34C open and valve 34B and 34D closed, a liquid runs down into tank 36. When tubes are fully coated with the solid, the flow of incoming melt is stopped and allowed to accumulate in buffer tank 29. The flow of coolant to the shell surrounding tubes 32 is replaced by water at a temperature such that the lowest-melting components of the coating on the tubes melt and flow into tank 36 or if desired into a separate tank (not shown). Such partial re-melting ("sweating") may be repeated one or more times at temperatures that are successively higher, but below that at which the coating melts to a major extent. The product of such later partial re-melts is fed into tank 38 (valves 34B and 34C open, valves 34B and 34D closed). Finally pressurised hot water at a temperature above the melting point of pure 4,4'-DCDPS is fed to the shell surrounding tubes 32, with valve 34D open and valves 34A, 34B and 34C closed; 4,4'-DCDPS is melted and fed out at 40 to stock or, if higher purity is required, to one or more further cycles of solidification, sweating and re-melting.

The contents of tank 36, which are relatively rich in CBSA, and of the tank (not shown) in which the first sweating product is collected, are recycled at 16 to reactor 26. The contents of tank 38, which are relatively rich in unwanted isomers of DCDPS, are passed to other users or discarded.

Example 1

In a preparation of purified 4,4'-DCDPS the flow rates, times and temperatures at significant positions in the flow sheet are calculated to be as in the Table. The flow rate is measured in kg per kg of product 4,4'-DCDPS.

EP 0 382 441 A2

Table

| Position | Temp °C | Flow Rate | | |
|---|---|---|---|---|
| | | DCDPS (all isomers) | CBSA | MCB |
| 10 outlet | 150 | 0.08 | 4.75 | 8.9 |
| 26 outlet | 190 | 3.2 | 3.3 | 1.3 |
| 36 outlet | 70 | 2.0 | 3.0 | 1.3 |
| 32 coating | 70 | 1.2 | 0.3 | 0.07 |
| 32 last melt | 70 | 1.0 | 0.0 | 0.0 |

Example 2

The following test run represents steps (b) and (c) of the process.

A mixture of CBSA with the 3 isomers of dichlorodiphenylsulphone was melted at 82.2°C and poured into a tube surrounded by coolant. Its temperature was lowered slowly at 35°C. Part of the charge, referred to below as 'residue', remained as a liquid phase and was taken off. The deposit on the surface was then warmed to 60°C over 4h, held at 60° for 1.5h, then warmed to 88.6°C over 3.5h. Partial melting took place. Samples of the melt were taken at intervals and analysed. The weight and composition of the feed and fractions taken at the various temperature stages are shown in Table 2.

Table 2

| Fraction | Temp. deg °C | Weight,g | Composition % w/w | | | |
|---|---|---|---|---|---|---|
| | | | CBSA | 4,4' | 3,4' | 2,4' |
| feed | 82.2 | 785 | 40 | 40.3 | 14.7 | 5 |
| residue | 35 | 173 | 57.6 | 19.7 | 16.7 | 6 |
| melt 1 | 44 | 104.6 | 55.5 | 23.5 | 15.5 | 5.5 |
| melt 2 | 62 | 95.3 | 52.4 | 25.8 | 16.0 | 5.8 |
| melt 3 | 78 | 98.8 | 48.0 | 33.1 | 14.1 | 4.8 |
| melt 4 | 88.6 | 57.1 | 41.1 | 40.9 | 13.5 | 4.5 |
| crystals | | 247.3 | 28.3 | 58.1 | 10.5 | 3.1 |

Claims

1. A process for producing an aromatic sulphone by reacting a sulphonic acid with an aromatic compound having at least one reactive position and recovering the sulphone from the reaction mixture, characterised by

a) providing a substantially anhydrous starting melt, optionally containing at most 18% w/w of volatile solvent, in which the relative concentrations of sulphonic acid and sulphone are on the sulphone-rich side of the relevant eutectic in the sulphonic acid/sulphone phase diagram;

b) cooling the melt to a temperature at which the sulphone crystallises and allowing it to crystallise;

c) separating the sulphone crystals and running off the supernatant liquid.

2. A process according to claim 1 in which the starting melt is provided by reacting the sulphonic acid

4

with the aromatic compound in water-removing conditions and controlling the reaction so that the relative concentrations of the unreacted sulphonic acid and sulphone are as defined in step (a).

3. A process according to claim 1 including the step of recycling said supernatant liquid whereby to recover unreacted sulphonic acid and possibly to convert unwanted isomers of the sulphonic to wanted isomers.

4. A process according to any one of claims 1 to 3 including at least once remelting the crystals from (c) and subjecting the melt to steps (b) and (c).

5. A process according to any one of claims 1 to 4 in which crystallisation step (b) is carried out by flowing the melt over a cooled surface, so that the crystals adhere to the surface, and periodically removing those crystals.

6. A process according to claim 5 which comprises removing the adherent crystals by re-melting, feeding the melt to a further heat exchanger and repeating steps (b) and (c) in that heat exchanger.

7. A process according to claim 5 which comprises re-melting the adherent crystals in at least 2 stages, taking off lower melting materials in stages at relatively low temperatures and recovering relatively pure sulphone at the highest temperature stage.

8. A process according to claim 7 in which the sulphonic acid is 4-chlorobenzenesulphonic acid, the aromatic compound is chlorobenzene and the sulphone recovered by crystallisation is 4,4′-dichlorodiphenylsulphone.

9. A process according to claim 8 which comprises remelting adherent crystals containing 4,4′-dichlorodiphenylsulphone at staged temperatures, taking fractions rich in 4-chlorobenzenesulphonic acid and/or the 2,4′ and 3,4′ isomers of dichlorodiphenylsulphone and recycling them and finally recovering relatively pure 4,4′-dichlorodiphenylsulphone.

10. A process for producing an aromatic sulphone which comprises

i) producing the corresponding aromatic sulphonic acid by sulphonating an aromatic compound;

ii) reacting the sulphonic acid with an aromatic compound having at least one reactive position in water-removing conditions and controlling the reaction so that the relative concentrations of the unreacted sulphonic acid and sulphone are on the sulphone-rich side of the relevant eutectic in the sulphonic acid/sulphone phase diagram;

iii) adjusting the composition of the product of step (ii) to provide a substantially anhydrous melt containing at most 18% w/w of volatile organic solvent; iv) cooling the melt to a temperature at which the sulphone crystallises and allowing it to crystallise;

v) separating the crystals enriched in sulphone and running off the supernatant liquid enriched in the sulphonic acid and in any unwanted isomers of the sulphone;

vi) in cascade at least once remelting the crystals and subjecting the melt to cooling and separation as in steps (iv) and (v);

vii) recovering purified sulphone as the crystals separated in the last operation of step (vi); and

viii) recycling to step (ii) supernatant liquids from steps (v) and (vi), whereby to utilise unreacted sulphonic acid and possibly to re-equilibrate unwanted sulphone isomers.

Fig.1(a).

SOLUBILITY CURVE: 4,4'—DCDPS/CBSA

BASIS: ——— SHFH (REGULAR SOLUTION ——— IDEALITY

*(graph axes: TEMPERATURE °C. vs 4,4'—DCDPS (MOL. FRACN.))*

EP 0 382 441 A2

Fig.1(b).

# Fig.2.